# EUROPÄISCHE PATENTSCHRIFT

(11) **EP 4 194 837 B1**
(45) Veröffentlichungstag und Bekanntmachung des Hinweises auf die Patenterteilung: **09.10.2024**
(21) Anmeldenummer: 21212872.2
(22) Anmeldetag: 07.12.2021
(51) Int. Cl.: G01N 15/00, A61M 11/00, B05B 7/00

(54) **AEROSOLGENERATOR UND VERFAHREN ZUR ABGABE EINES AEROSOLS**
AEROSOL GENERATOR AND AEROSOL DELIVERY METHOD
GÉNÉRATEUR D'AÉROSOL ET PROCÉDÉ DE DISTRIBUTION D'UN AÉROSOL

(43) Veröffentlichungstag der Anmeldung: 14.06.2023
(73) Patentinhaber: Fraunhofer-Gesellschaft zur Förderung der angewandten Forschung e.V., 80686 München (DE)
(72) Erfinder: NORREFELDT, Victor, 83626 Valley (DE); BUSCHHAUS, Michael, 83626 Valley (DE); NAGELE-RENZL, Anna, 83626 Valley (DE)
(74) Vertreter: Friese Goeden Patentanwälte PartGmbB

(56) Entgegenhaltungen:
- DE-U1- 202015 105 630
- SVENSSON MÅRTEN ET AL: "Laboratory Study Comparing Pharmacopeial Testing of Nebulizers with Evaluation Based on Nephele Mixing Inlet Methodology", AAPS PHARMSCITECH, SPRINGER US, NEW YORK, vol. 19, no. 2, 5 September 2017 (2017-09-05), pages 565 - 572, XP036412864, DOI: 10.1208/S12249-017-0860-8
- DAVID KRISTO ET AL: "The potential for delivery of particulate matter through positive airway pressure devices (CPAP/BPAP)", SLEEP AND BREATHING ; INTERNATIONAL JOURNAL OF THE SCIENCE AND PRACTICE OF SLEEP MEDICINE, SPRINGER, BERLIN, DE, vol. 16, no. 1, 16 January 2011 (2011-01-16), pages 193 - 198, XP035013931, ISSN: 1522-1709, DOI: 10.1007/S11325-010-0475-8

## Beschreibung

Die Erfindung betrifft einen Aerosolgenerator zur Abgabe eines Aerosols in einen Raum, mit einem Vorratsbehälter zur Aufnahme einer Flüssigkeit, einer Vernebelungseinrichtung zur Erzeugung eines Aerosols aus der Flüssigkeit und einer ersten Leitung, welche die Vernebelungseinrichtung und/oder den Vorratsbehälter mit zumindest einer Austrittsöffnung verbindet, wobei die Austrittsöffnung dazu eingerichtet ist, das Aerosol in den Raum abzugeben. Weiterhin betrifft die Erfindung ein Verfahren zur Abgabe eines Aerosols in einen Raum. Solche Vorrichtungen und Verfahren können zur Erforschung der Ausbreitung von Aerosolen in einem Raum eingesetzt werden. Siehe auch Dokument: SVENSSON MARTEN ET AL: "Laboratory Study Comparing Pharmacopeia! Testing of Nebulizers with Evaluation Based on Nephele Mixing Inlet Methodology", AAPS PHARMSCITECH, SPRINGER US, NEW YORK, Bd. 19, Nr. 2, 5. September 2017 (2017-09-05), Seiten 565-572.

Aus der DE 20 2015 105 630 U1 ist ein Aerosolgenerator bekannt, der mittels einer Zweistoffdüse eine Flüssigkeit zerstäuben kann, um Prüfaerosole bereitzustellen. Dieser Aerosolgenerator weist jedoch den Nachteil auf, dass die Ausblasrichtung der erzeugten Aerosole für die menschliche Atmung nicht repräsentativ ist. Untersuchungen zur Ausbreitung von Bioaerosolen in einem Raum können daher nicht mit hoher Genauigkeit durchgeführt werden.

Ausgehend vom Stand der Technik liegt der Erfindung somit die Aufgabe zugrunde, einen Aerosolgenerator und ein Verfahren zur Abgabe eines Aerosols bereitzustellen, welche Messungen zur Ausbreitung von Aerosolen mit hoher Genauigkeit ermöglichen, wenn diese Aerosole von Menschen oder Tieren verbreitet werden.

Die Aufgabe wird erfindungsgemäß durch eine Vorrichtung gemäß Anspruch 1 und ein Verfahren nach Anspruch 9 gelöst. Vorteilhafte Weiterbildungen der Erfindung finden sich in den Unteransprüchen.

Erfindungsgemäß wird ein Aerosolgenerator zur Abgabe eines Aerosols in einen Raum vorgeschlagen. Der Raum kann beispielsweise ein Wohnraum, ein Büro oder ein Veranstaltungsraum sein. In anderen Ausführungsformen der Erfindung kann der Raum das Innere eines Verkehrsmittels sein, beispielsweise ein Fahrzeug, ein Flugzeug, ein Schiff, eine Aufzugkabine oder eine Seilbahngondel. Der Aerosolgenerator erlaubt die Abgabe eines Prüf- bzw. Testaerosols in diesen Raum, sodass dessen Ausbreitung innerhalb des Raums messtechnisch erfasst werden kann. Eine solche Messung kann bei der Auslegung bzw. Optimierung einer Lüftungs- oder Klimaanlage oder einem Raumluftreiniger hilfreich sein oder die Entwicklung einer Strategie zur Fensterlüftung ermöglichen oder zumindest unterstützen.

Der erfindungsgemäße Aerosolgenerator ist mit einem Vorratsbehälter ausgestattet, in welchen bei Betrieb der Vorrichtung eine Flüssigkeit aufgenommen wird. Die Flüssigkeit kann beispielsweise Wasser oder eine wässrige Lösung enthalten oder daraus bestehen. Beispielsweise kann die Flüssigkeit eine Salzlösung sein oder einen Marker enthalten, welcher in einfacher Weise messtechnisch erfasst werden kann und so die spätere Ausbreitung des Aerosols nachvollziehbar macht. In einigen Ausführungsformen der Erfindung kann die Flüssigkeit im Vorratsbehälter eine Pufferlösung sein bzw. eine Pufferlösung enthalten, welche Ihrerseits eine Mischung aus verschiedenen Salzen und organischen und/oder anorganischen Substanzen enthält. In einigen Ausführungsformen der Erfindung kann die Flüssigkeit als Marker Bakteriophagen enthalten. Solche Bakteriophagen weisen als Viren ein ähnliches bzw. vergleichbares Ausbreitungsverhalten auf wie pathogene Keime, sind jedoch für Menschen oder Tiere unschädlich. In anderen Ausführungsformen der Erfindung kann die Flüssigkeit zumindest ein Öl oder ein Liposom enthalten oder daraus bestehen.

Darüber hinaus weist der erfindungsgemäße Aerosolgenerator eine Vernebelungseinrichtung auf, welche dazu eingerichtet ist, bei Betrieb des Aerosolgenerators aus der Flüssigkeit im Vorratsbehälter ein Aerosol zu erzeugen. Das Aerosol kann einzelne Tröpfchen bzw. Partikel enthalten, welche einen Durchmesser von etwa 5 nm µm bis etwa 30 µm oder von etwa 2,5 µm bis etwa 20 µm oder von etwa 1 µm bis etwa 10 µm oder von etwa 5 nm bis etwa 1 µm oder von etwa 1 µm bis etwa 2,5 µm aufweisen. Die Größe der einzelnen Tröpfchen des Aerosols kann in einigen Ausführungsformen der Erfindung so gewählt sein, dass diese repräsentativ für die während der Atmung von Menschen ausgestoßenen Aerosole ist.

Der Aerosolgenerator verfügt weiterhin über zumindest eine Austrittsöffnung, welche dazu vorgesehen ist, die von der Vernebelungseinrichtung erzeugten Aerosole in den Raum abzugeben. Zwischen dem Vorratsbehälter bzw. der Vernebelungseinrichtung und der Austrittsöffnung befindet sich eine erste Leitung, welche das Aerosol vom Ort seiner Entstehung zur Austrittsöffnung transportiert. Hierzu kann die erste Leitung eine Schlauch- oder Rohrleitung sein, beispielsweise mit einem Nenndurchmesser von etwa 10 mm bis etwa 50 mm oder von etwa 15 mm bis etwa 40 mm.

Erfindungsgemäß wird nun vorgeschlagen, dass der Aerosolgenerator weiterhin zumindest eine Luftfördereinrichtung enthält, welche dazu eingerichtet ist, die Strömungsrichtung an der Austrittsöffnung zyklisch umzukehren. Die Luftströmung an der Austrittsöffnung simuliert somit den Atemzyklus eines Menschen bzw. eines Tieres, indem durch die Luftfördereinrichtung die Luft zyklisch ausgestoßen und eingesaugt wird. Dementsprechend werden auch die Aerosole zyklisch ausgestoßen, sodass die Ausströmgeschwindigkeit an der Austrittsöffnung nicht konstant ist. Die Luftströmung und der zeitliche Verlauf des Ausstoßes des Aerosols kommt somit derjenigen Luftströmung nahe, welche an den Atemöffnungen von Lebewesen beobachtet werden kann. Um dies zu ermöglichen, wird die Luftfördereinrichtung mittels zumindest einer zweiten Leitung mit der ersten Leitung verbunden, sodass durch zyklische Druckerhöhung und Druckerniedrigung die gewünschte zyklische Luftströmung an der Austrittsöffnung entsteht.

Der erfindungsgemäße Aerosolgenerator erlaubt somit anders als bekannte Geräte einen zyklischen Aerosolausstoß mit wechselnder Strömungsgeschwindigkeit, sowie dieser auch natürlicherweise an den Atemöffnungen von Lebewesen beobachtet werden kann. Der Einfluss der natürlichen Atmung auf die Aerosolausbreitung in einem Raum kann somit mit dem erfindungsgemäßen Aerosolgenerator mit hoher Genauigkeit simuliert und gemessen werden. Bei der Auslegung oder Überprüfung von Lüftungs- oder Klimaanlagen oder einem Raumluftreiniger oder Strategien zur Fensterlüftung können somit genauere Messung ermöglicht werden.

In einigen Ausführungsformen der Erfindung kann die Austrittsöffnung Teil eines Prüfkopfes mit einer dem menschlichen Kopf angenäherten Form sein. Der Prüfkopf kann wiederum auf einem Stativ montiert oder Teil einer menschlichen Puppe sein, sodass die Aerosole in einer vergleichbaren Höhe und in einer vergleichbare Richtung ausgestoßen werden, wie dies auch bei menschlichen Probanden der Fall wäre. Die Austrittsöffnung kann in diesem Fall beispielsweise eine Mund- und/oder Nasenöffnung sein bzw. eine solche simulieren. Sofern der Prüfkopf Teil einer Puppe ist, kann diese durch weitere technische Einrichtungen weitere Emissionen verursachen, welche die Qualität der Raumluft beeinträchtigen, beispielsweise CO₂ und/oder Wärme. In einigen Ausführungsformen der Erfindung ist die Vernebelungseinrichtung ausgewählt aus einem Piezovernebler oder einem Düsenvernebler oder einem Membranvernebler. Die Vernebelungseinrichtung kann dazu eingerichtet sein, Aerosole mit einem Partikel- bzw. Tropfendurchmesser von etwa 5 nm bis etwa 20 µm bereitzustellen. Die Vernebelungseinrichtung kann eine Flüssigkeitszufuhr aufweisen, über welche die Flüssigkeit aus dem Vorratsbehälter angesaugt und als Aerosol über einen Auslass der Vernebelungseinrichtung abgegeben wird. In anderen Ausführungsformen der Erfindung kann die Vernebelungseinrichtung innerhalb des Vorratsbehälters angeordnet sein, sodass das Aerosol unmittelbar aus dem Vorratsbehälter ausgetragen wird. Die Vernebelungseinrichtung kann optional mit einer Steuer- oder Regeleinrichtung versehen sein, welche die Menge des erzeugten Aerosols und/oder die Größenverteilung der einzelnen Tröpfchen bzw. Partikel des Aerosols beeinflusst.

In einigen Ausführungsformen der Erfindung ist in der zweiten Leitung ein erstes Rückschlagventil angeordnet. Dieses verhindert, dass in der Ansaugphase der alternierenden Luftströmung das Aerosol in die Luftfördereinrichtung gelangt.

In einigen Ausführungsformen der Erfindung enthält der Aerosolgenerator weiterhin eine dritte Leitung mit einem zweiten Rückschlagventil, welches parallel und mit entgegengesetzter Durchflussrichtung zum ersten Rückschlagventil in der dritten Leitung angeordnet ist. Diese ermöglicht es, einen Kurzschluss der Strömung in der Luftfördereinrichtung zu verhindern, sodass die von der Luftfördereinrichtung geförderte bzw. angesaugte Luft bis auf ein geringes Totvolumen in den Leitungen durch die Austrittsöffnung austreten kann.

In einigen Ausführungsformen der Erfindung kann die Luftfördereinrichtung eine Kolbenpumpe sein, welche bei alternierender Bewegung des Kolben Luft abwechselnd ausstößt und ansaugt, um auf diese Weise die gewünschte alternierende Luftströmung an der Austrittsöffnung des Aerosolgenerators zu erzeugen.

Die Rückschlagventile können in einigen Ausführungsformen der Erfindung federbelastete Tellerventile sein, welche aufgrund ihrer Bauform einseitig durchströmbar sind und einseitig dichten. In anderen Ausführungsformen der Erfindung können auch elektrisch oder pneumatisch schaltende Durchflussventile eingesetzt werden, welche durch eine Steuer- oder Regeleinrichtung so angesteuert werden, dass diese nur dann geöffnet sind, wenn eine Strömung in der gewünschten Richtung anliegt.

In einigen Ausführungsformen der Erfindung kann die Luftfördereinrichtung dazu eingerichtet sein, die Strömungsrichtung an der Austrittsöffnung etwa 8-mal pro Minute bis etwa 50-mal pro Minute zyklisch umzukehren. Diese Frequenzen entsprechen im Wesentlichen den Frequenzen menschlicher Atmung bei geringer bzw. höherer körperlicher Beanspruchung, sodass die Aerosolausbreitung für eine Vielzahl unterschiedlicher Szenarien experimentell simuliert werden kann.

In einigen Ausführungsformen der Erfindung kann die Luftfördereinrichtung dazu eingerichtet sein, mit jedem zyklischen Arbeitstakt ein Luftvolumen von etwa 600 ml bis etwa 4000 ml zu fördern. In anderen Ausführungsformen der Erfindung kann die Luftfördereinrichtung dazu eingerichtet sein, mit jedem zyklischen Arbeitstakt ein Luftvolumen von etwa 1500 ml bis etwa 3000 ml zu fördern. In wiederum anderen Ausführungsformen der Erfindung kann die Luftfördereinrichtung dazu eingerichtet sein, mit jedem zyklischen Arbeitstakt ein Luftvolumen von etwa 700 ml bis etwa 1500 ml zu fördern. Die genannten Luftvolumina entsprechen in etwa der Luftmenge, welche von einer erwachsenen Person pro Atemzug aus- bzw. eingeatmet wird, sodass die Aerosolverteilung des Aerosolgenerators der menschlichen Atmung möglichst nahe kommt.

In einigen Ausführungsformen der Erfindung ist die Luftfördereinrichtung dazu eingerichtet, in der Austrittsöffnung eine maximale Strömungsgeschwindigkeit von etwa 0,5 m/s bis etwa 2 m/s zu erzeugen. In anderen Ausführungsformen der Erfindung kann die Luftfördereinrichtung dazu eingerichtet sein, in der Austrittsöffnung eine maximale Strömungsgeschwindigkeit von etwa 0,8 m/s bis etwa 1,8 m/s zu erzeugen. In wiederum anderen Ausführungsformen kann die Luftfördereinrichtung dazu eingerichtet sein, in der Austrittsöffnung eine maximale Strömungsgeschwindigkeit von etwa 1,3 m/s bis etwa 1,5 m/s zu erzeugen. Diese Strömungsgeschwindigkeiten entsprechen der physiologischen Atmung eines Menschen, sodass der hierdurch bedingte Aerosolaustrag des Aerosolgenerators mit nur geringen Fehlern dem Aerosolaustrag eines Menschen entspricht.

Nachfolgend soll die Erfindung anhand von Figuren ohne Beschränkung des allgemeinen Erfindungsgedankens näher erläutert werden. Dabei zeigt
Figur 1 ein Blockschaltbild des erfindungsgemäßen Aerosolgenerators.
Figur 2 zeigt die Strömungsgeschwindigkeit an der Austrittsöffnung gegen die Zeit für ein erstes Ausführungsbeispiel.
Figur 3 zeigt die gemessene Partikelgrößenverteilung gegen den Abstand von der Austrittsöffnung für das erste Ausführungsbeispiel.
Figur 4 zeigt die gemessene Partikelgrößenverteilung gegen den Abstand von der Austrittsöffnung für ein zweites Ausführungsbeispiel.

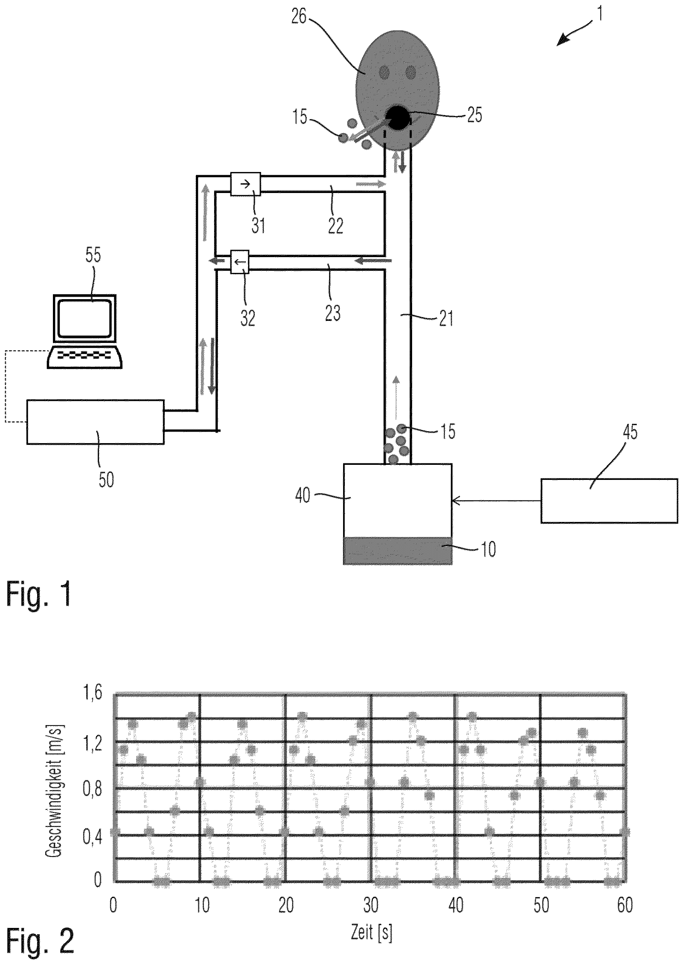

Anhand der Figur 1 wird ein erfindungsgemäßer Aerosolgenerator 1 zur Abgabe eines Aerosols 15 in einen Raum näher erläutert. Der Raum kann beispielsweise der Innenraum eines Verkehrsmittels, ein Wohnraum, ein Büro oder eine Werkstatt, ein Stall, eine Sporthalle oder ein Veranstaltungsraum sein. In diesem Raum soll die Ausbreitung von Aerosolen messtechnisch bestimmt werden, welche durch Menschen oder Tiere in den Raum eingetragen werden.

Hierzu wird im Raum zumindest ein Aerosolgenerator 1 aufgestellt. Selbstverständlich kann auch eine größere Anzahl von Aerosolgeneratoren aufgestellt werden, wenn eine größere Besetzung des Raumes simuliert werden soll, beispielsweise im Falle eines Veranstaltungsortes, eines Seminarraumes oder eines Klassenzimmers.

Der Aerosolgenerator 1 enthält einen Vorratsbehälter 40 zur Aufnahme einer Flüssigkeit 10. Die Flüssigkeit 10 ist dazu eingerichtet und bestimmt, mittels einer Vernebelungseinrichtung 45 in ein Aerosol 15 überführt zu werden. Die Vernebelungseinrichtung 45 kann beispielsweise ausgewählt sein aus einem Piezovernebler, einem Düsenvernebler oder einem Membranvernebler. Die Vernebelungseinrichtung 45 kann dazu eingerichtet sein, Partikel bzw. Tröpfen mit einem Durchmesser von etwa 5 nm bis etwa 20 µm oder von etwa 10 nm bis etwa 10 µm zu erzeugen.

Der Vorratsbehälter 40 bzw. die Vernebelungseinrichtung 45 ist mittels einer ersten Leitung 21 mit einer Austrittsöffnung 25 verbunden, durch welche das Aerosol 15 in den Raum abgegeben wird.

Die Austrittsöffnung 25 ist Teil eines Prüfkopfes 26, welcher eine dem menschlichen Kopf angenäherten Form hat. Im dargestellten Ausführungsbeispiel ist nur eine einzige Austrittsöffnung 25 als Mundöffnung dargestellt. In anderen Ausführungsformen der Erfindung kann der Prüfkopf auch eine Mehrzahl von Austrittsöffnungen haben, sodass das Aerosol 15 beispielsweise auch durch Nasenöffnungen abgegeben werden kann, welche beispielsweise einen geringeren Querschnitt und/oder eine andere Ausströmrichtung aufweisen wie die Mundöffnung. Die Austrittsöffnung 25 ist insofern nur erläuternd und nicht beschränkend zu sehen.

Die Vernebelungseinrichtung 45 gibt das Aerosol 15 kontinuierlich ab, was indes nicht dem natürlichen Atemverhalten von Lebewesen entspricht. Vielmehr dreht sich die Strömungsrichtung an den Atemöffnungen von Lebewesen zyklisch um, d. h. Einatmung und Ausatmung wechseln sich periodisch ab. Zur Simulation dieses Strömungsverhaltens steht eine Luftfördereinrichtung 50 zur Verfügung, welche mit einer zweiten Leitung 22 und einer dritten Leitung 23 mit der ersten Leitung 21 verbunden ist und über die erste Leitung 21 auch mit der Austrittsöffnung 25 in Kontakt steht.

Die Luftfördereinrichtung 50 ist dazu eingerichtet und bestimmt, eine Luftströmung zu erzeugen, welche ihre Richtung zyklisch umkehrt. Beispielsweise kann die Luftfördereinrichtung hierzu eine Kolbenpumpe enthalten oder daraus bestehen. Die Luftfördereinrichtung kann dazu eingerichtet sein, die Strömungsrichtung an der Austrittsöffnung 25 etwa 8-mal pro Minute bis etwa 50-mal pro Minute zyklisch umzukehren bzw. etwa 8 bzw. bis etwa 50 Atemzüge pro Minute simulieren. Bei jedem dieser zyklischen Arbeitstakte bzw. simulierten Atemzüge kann die Luftfördereinrichtung ein Luftvolumen von etwa 600 ml bis etwa 4000 ml bewegen. In anderen Ausführungsformen der Erfindung kann das Luftvolumen pro Arbeitstakt etwa 1500 ml bis etwa 3000 ml oder etwa 700 ml bis etwa 1500 ml betragen. Durch Anpassung des Luftvolumens und der Anzahl der Arbeitstakte pro Minute der Luftfördereinrichtung kann somit die Atmung in Ruhe oder bei größerer körperlicher Belastung simuliert werden. Zur Ansteuerung der Luftfördereinrichtung 50 und/oder der Vernebelungseinrichtung 45 kann eine Steuer- oder Regeleinrichtung 55 vorhanden sein. Diese kann beispielsweise aus einem handelsüblichen PC bestehen bzw. einen solchen enthalten, auf welchem eine zur Ansteuerung der Luftfördereinrichtung 50 und/oder der Vernebelungseinrichtung 45 bestimmte Software ausgeführt wird. In gleicher Weise kann die Steuer- oder Regeleinrichtung auch alternativ oder zusätzlich einen Mikroprozessor oder einen Mikrocontroller enthalten oder als diskrete elektronische Schaltung aufgebaut sein.

In der zweiten Leitung 22 und der dritten Leitung 23 befindet sich jeweils ein erstes und zweites Rückschlagventil 31 und 32, sodass die von der Luftfördereinrichtung 50 ausgestoßene Luft durch das erste Rückschlagventil 31 und die zweite Leitung 22 in die erste Leitung 21 und die Austrittsöffnung 25 strömt. Zur Simulation des Einatmens strömt die Luft aus dem Raum durch die Austrittsöffnung 25 in die erste Leitung 21 und von dort über die dritte Leitung 23 und das zweite Rückschlagventil 32 zur Luftfördereinrichtung 50.

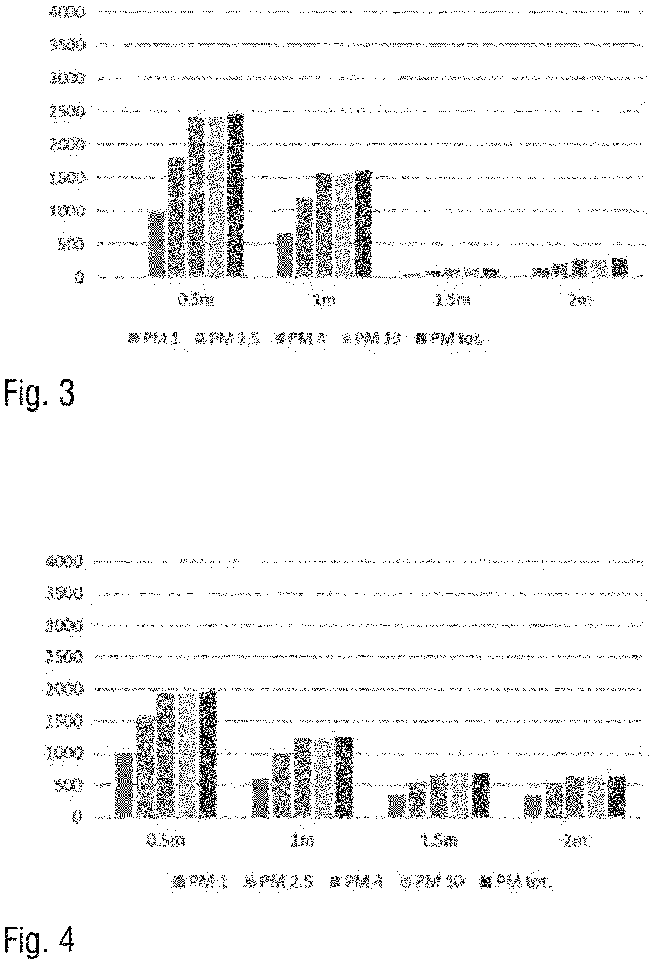

Anhand der Figuren 2 bis 4 werden nachfolgend Messergebnisse präsentiert, welche mit dem erfindungsgemäßen Aerosolgenerator erhalten wurden. Fig. 2 und 3 zeigen Daten eines ersten Ausführungsbeispiels, bei welchem die Luftfördereinrichtung 50 so eingestellt wurde, dass diese pro Arbeitstakt bzw. Atemzug 0,715 Liter Luftvolumen fördert, wobei neun Arbeitstakte bzw. Atemzüge pro Minute simuliert werden. Fig. 4 zeigt Daten eines zweiten Ausführungsbeispiels, bei welchem die Luftfördereinrichtung 50 so eingestellt wurde, dass diese pro Arbeitstakt bzw. Atemzug 2 Liter Luftvolumen fördert, wobei 39 Arbeitstakte bzw. Atemzüge pro Minute simuliert werden

Figur 2 zeigt die Strömungsgeschwindigkeit auf der Ordinate gegen die Zeit auf der Abszisse, wobei sich ein etwa sinusförmiger Verlauf des Ausatmens mit einem Maximalwert von etwa 1,4 m/s zeigt. Zu Beginn und am Ende des Ausatmens sinkt die Geschwindigkeit auf 0 m/s ab. Beim Einatmen kann keine Strömungsgeschwindigkeit an der Austrittsöffnung gemessen werden, weil die Raumluft von allen Seiten angesaugt wird.

Es hat sich gezeigt, dass im ersten Ausführungsbeispiel in einem Abstand von etwa 0,5 m vor der Austrittsöffnung 25 keine Strömungsgeschwindigkeit der Raumluft detektierbar ist. Simuliert man gemäß dem zweiten Ausführungsbeispiel eine größere körperliche Belastung, reicht die Strömung ausgehend von der Austrittsöffnung 25 etwa 0,5 m in den Raum hinein.

Anhand der Figuren 3 und 4 wird die Partikelverteilung in Abständen von 0,5 m, 1 m, 1,5 m und 2 m von der Austrittsöffnung 25 dargestellt. Dabei zeigt Figur 3 die gemessene Partikelverteilung für den Fall eines Atemvolumens von 715 ml bei neun Atemzügen pro Minute und Figur 4 zeigt die gemessene Partikelverteilung für ein Atemvolumen von 2000 ml pro Atemzug bei 39 Atemzügen pro Minute. Dargestellt sind jeweils die Anzahl der Partikel gegen die Partikelgrößen für Partikel ≤ 1 µm, für Partikel ≤ 2,5 µm, für Partikel ≤ 4 µm und für Partikel ≤ 10 µm. Der letzte Messwert zeigt die Gesamtzahl der bestimmten Partikel unabhängig von ihrer Größe an.

Der Vergleich der Figuren 3 und 4 zeigt, dass in Ruhe, d. h. bei geringem Atemvolumen, eine stark lokalisierte Aerosolwolke um den Aerosolgenerator erzeugt wird. Bereits im Abstand von etwa 1,5 m nimmt die Partikelkonzentration stark ab. Bei größerer körperlicher Belastung reicht die ausgestoßene Aerosolwolke hingegen weiter in den Raum hinein, sodass auch in einem Abstand von 2 m noch eine deutliche Partikelkonzentration gemessen wird. Somit erlaubt der erfindungsgemäße Aerosolgenerator und das erfindungsgemäße Verfahren zu dessen Betrieb erstmalig, eine genaue Simulation des Aerosolausstoßes eines Lebewesens, um die Partikelverteilung unter realen Bedingungen messtechnisch zu erfassen. Hierdurch können beispielsweise Lüftungs- oder Klimaanlagen oder Raumluftreiniger oder auch Strategien zur manuellen Fensterlüftung optimiert werden.

## Patentansprüche

1. Aerosolgenerator (1) zur Abgabe eines Aerosols (15) in einen Raum, mit
einem Vorratsbehälter (40) zur Aufnahme einer Flüssigkeit (10),
einer Vernebelungseinrichtung (45) zur Erzeugung eines Aerosols (15) aus der Flüssigkeit (10),
einer ersten Leitung (21), welche die Vernebelungseinrichtung (45) und/oder den Vorratsbehälter (40) mit zumindest einer Austrittsöffnung (25) verbindet, wobei die Austrittsöffnung (25) dazu eingerichtet ist,das Aerosol (15) in den Raum abzugeben,
**dadurch gekennzeichnet, dass**
der Aerosolgenerator (1) weiterhin eine Luftfördereinrichtung (50) enthält, welche dazu eingerichtet ist, die Strömungsrichtung an der Austrittsöffnung (25) zyklisch umzukehren, wobei
die Luftfördereinrichtung (50) mittels zumindest einer zweiten Leitung (22) mit der ersten Leitung (21) verbunden ist und in der zweiten Leitung (22) ein erstes Rückschlagventil (31) vorhanden ist.

2. Aerosolgenerator nach Anspruch 1, **dadurch gekennzeichnet, dass** die Austrittsöffnung (25) Teil eines Prüfkopfes (26) mit einer dem menschlichen Kopf angenäherten Form ist.

3. Aerosolgenerator nach Anspruch 1 oder 2, **dadurch gekennzeichnet, dass** die Vernebelungseinrichtung (45) ausgewählt ist aus einem Piezovernebler oder einem Düsenvernebler oder einem Membranvernebler.

4. Aerosolgenerator nach einem der Ansprüche 1 bis 3, weiterhin enthaltend eine dritte Leitung (23) mit einem zweiten Rückschlagventil (32), welches parallel und mit entgegengesetzter Durchflussrichtung zum ersten Rückschlagventil (31) in der dritten Leitung (23) angeordnet ist.

5. Aerosolgenerator nach einem der Ansprüche 1 bis 4, **dadurch gekennzeichnet, dass** die Luftfördereinrichtung (50) dazu eingerichtet ist, die Strömungsrichtung an der Austrittsöffnung (25) etwa 8 mal pro Minute bis etwa 50 mal pro Minute zyklisch umzukehren.

6. Aerosolgenerator nach einem der Ansprüche 1 bis 5, **dadurch gekennzeichnet, dass** die Luftfördereinrichtung (50) dazu eingerichtet ist, mit jedem zyklischen Arbeitstakt ein Luftvolumen von etwa 600 ml bis etwa 4000 ml oder von etwa 1500 ml bis etwa 3000 ml oder von etwa 700 ml bis etwa 1500 ml zu fördern.

7. Aerosolgenerator nach einem der Ansprüche 1 bis 6, **dadurch gekennzeichnet, dass** die Luftfördereinrichtung (50) dazu eingerichtet ist, in der Austrittsöffnung (25) eine maximale Strömungsgeschwindigkeit von etwa 0,5 m/s bis etwa 2 m/s oder von etwa 0,8 m/s bis etwa 1,8 m/s oder von etwa 1,3 m/s bis etwa 1,5 m/s zu erzeugen.

8. Verfahren zur Abgabe eines Aerosols in einen Raum, mit folgenen Schritten:
bereitstellen eines Vorratsbehälters (40) mit einer Flüssigkeit (10),
Erzeugung eines Aerosols (15) aus der Flüssigkeit (10) mit einer Vernebelungseinrichtung (45),
Abgabe des Aerosols (15) über eine erste Leitung (21) und eine Austrittsöffnung (25) in den Raum,
**dadurch gekennzeichnet, dass**
die Strömungsrichtung an der Austrittsöffnung (25) zyklisch umgekehrt wird, indem eine Luftfördereinrichtung (50), welche mittels zumindest einer zweiten Leitung (22) mit der ersten Leitung (21) verbunden ist, zyklisch Luft fördert und ansaugt, wobei in der zweiten Leitung (22) ein erstes Rückschlagventil (31) vorhanden ist.

9. Verfahren nach Anspruch 8, **dadurch gekennzeichnet, dass** das Aerosol (15) aus der Flüssigkeit (10) mit einem Piezovernebler oder einem Düsenvernebler oder einem Membranvernebler erzeugt wird.

10. Verfahren nach einem der Ansprüche 8 oder 9, **dadurch gekennzeichnet, dass** die Strömungs¬richtung an der Austrittsöffnung (25) etwa 8 mal pro Minute bis etwa 50 mal pro Minute zyklisch umgekehrt wird.

11. Verfahren nach einem der Ansprüche 8 bis 10, **dadurch gekennzeichnet, dass** mit jedem zyklischen Arbeitstakt ein Luftvolumen von etwa 600 ml bis etwa 4000 ml oder von etwa 1500 ml bis etwa 3000 ml oder von etwa 700 ml bis etwa 1500 ml durch die Austrittsöffnung (25) gefördert wird.

12. Verfahren nach einem der Ansprüche 8 bis 11, **dadurch gekennzeichnet, dass** in der Austrittsöffnung (25) eine maximale Strömungsgeschwindigkeit von etwa 0,5 m/s bis etwa 2 m/s oder von etwa 0,8 m/s bis etwa 1,8 m/s oder von etwa 1,3 m/s bis etwa 1,7 m/s erzeugt wird.

13. Verfahren nach einem der Ansprüche 8 bis 12, **dadurch gekennzeichnet, dass** die Flüssigkeit (10) im Vorratsbehälter (40) Wasser oder ein Öl oder ein Liposom enthält oder daraus besteht und/oder
dass die Flüssigkeit (10) im Vorratsbehälter (40) eine Pufferlösung enthält oder daraus besteht.

14. Verfahren nach Anspruch 13, **dadurch gekennzeichnet, dass** die Flüssigkeit (10) im Vorratsbehälter (40) Bakteriophagen enthält.

## Claims

1. Aerosol generator (1) for delivering an aerosol (15) into a room, comprising
a storage container (40) for accommodating a liquid (10),
a nebulization device (45) for generating an aerosol (15) from the liquid (10),
a first line (21) which connects the nebulization device (45) and/or the storage container (40) to at least one outlet opening (25), the outlet opening (25) being designed to deliver the aerosol (15) into the room,
**characterized in that**
the aerosol generator (1) further contains an air conveying device (50) which is designed to cyclically reverse the direction of flow at the outlet opening (25),
the air conveying device (50) being connected to the first line (21) by means of at least a second line (22) and a first check valve (31) being present in the second line (22).

2. Aerosol generator according to claim 1, **characterized in that** the outlet opening (25) is part of a test head (26) having a form approximating that of a human head.

3. Aerosol generator according to claim 1 or 2, **characterized in that** the nebulization device (45) is selected from a piezo nebulizer or a nozzle nebulizer or a membrane nebulizer.

4. Aerosol generator according to any one of claims 1 to 3, further comprising a third line (23) having a second check valve (32), which is arranged in parallel and in opposite flow direction to the first check valve (31) in the third line (23).

5. Aerosol generator according to any one of claims 1 to 4,
**characterized in that** the air conveying device (50) is designed to cyclically reverse the flow direction at the outlet opening (25) about 8 times per minute to about 50 times per minute.

6. Aerosol generator according to any one of claims 1 to 5, **characterized in that** the air conveying device (50) is designed to convey an air volume from about 600 ml to about 4000 ml or from about 1500 ml to about 3000 ml or from about 700 ml to about 1500 ml with each cyclic work cycle.

7. Aerosol generator according to any one of claims 1 to 6, **characterized in that** the air conveying device (50) is designed to generate a maximum flow velocity from about 0.5 m/s to about 2 m/s or from about 0.8 m/s to about 1.8 m/s or from about 1.3 m/s to about 1.5 m/s in the outlet opening (25).

8. Method for delivering an aerosol into a room, comprising the following steps:
providing a storage container (40) with a liquid (10),
generating an aerosol (15) from the liquid (10) with a nebulization device (45),
delivering the aerosol (15) via a first line (21) and an outlet opening (25) into the room,
**characterized in that**
the flow direction at the outlet opening (25) is cyclically reversed **in that** an air conveying device (50), which is connected to the first line (21) by means of at least a second line (22), cyclically conveys and draws in air, a first check valve (31) being present in the second line (22).

9. Method according to claim 8, **characterized in that** the aerosol (15) is produced from the liquid (10) by means of a piezo nebulizer or a nozzle nebulizer or a membrane nebulizer.

10. Method according to any one of claims 8 or 9, **characterized in that** the flow direction at the outlet opening (25) is cyclically reversed about 8 times per minute to about 50 times per minute.

11. Method according to any one of claims 8 to 10, **characterized in that** with each cyclic work cycle an air volume from about 600 ml to about 4000 ml or from about 1500 ml to about 3000 ml or from about 700 ml to about 1500 ml is conveyed through the outlet opening (25).

12. Method according to any one of claims 8 to 11, **characterized in that** a maximum flow velocity from about 0.5 m/s to about 2 m/s or from about 0.8 m/s to about 1.8 m/s or from about 1.3 m/s to about 1.7 m/s is generated in the outlet opening (25).

13. Method according to any one of claims 8 to 12, **characterized in that** the liquid (10) in the storage container (40) contains or consists of water or an oil or a liposome and/or
**in that** the liquid (10) in the storage container (40) contains or consists of a buffer solution.

14. Method according to claim 13, **characterized in that** the liquid (10) in the storage container (40) contains bacteriophages.

## Revendications

1. Générateur d'aérosol (1) pour distribuer un aérosol (15) dans un local, comprenant
un réservoir (40) destiné à recevoir un liquide (10),
un dispositif de nébulisation (45) pour produire un aérosol (15) à partir du liquide (10),
une première conduite (21) qui relie le dispositif de nébulisation (45) et/ou le réservoir (40) à au moins une ouverture de sortie (25), l'ouverture de sortie (25) étant conçue pour distribuer l'aérosol (15) dans le local,
**caractérisé en ce que**
le générateur d'aérosol (1) comprend en outre un dispositif de transport d'air (50) conçu pour inverser de manière cyclique le sens d'écoulement au niveau de l'ouverture de sortie (25),
le dispositif de transport d'air (50) étant relié à la première conduite (21) au moyen d'au moins une deuxième conduite (22), et un premier clapet anti-retour (31) étant prévu dans la deuxième conduite (22).

2. Générateur d'aérosol selon la revendication 1,
**caractérisé en ce que** l'ouverture de sortie (25) fait partie d'une tête de contrôle (26) dont la forme se rapproche de celle d'une tête humaine.

3. Générateur d'aérosol selon la revendication 1 ou 2,
**caractérisé en ce que** le dispositif de nébulisation (45) est choisi parmi un nébuliseur piézoélectrique ou un nébuliseur à buse ou un nébuliseur à membrane.

4. Générateur d'aérosol selon l'une des revendications 1 à 3,
comprenant en outre une troisième conduite (23) qui comporte un deuxième clapet anti-retour (32) disposé dans la troisième conduite (23) parallèlement au premier clapet anti-retour (31) et avec un sens d'écoulement opposé à celui-ci.

5. Générateur d'aérosol selon l'une des revendications 1 à 4,
**caractérisé en ce que** le dispositif de transport d'air (50) est conçu pour inverser de manière cyclique le sens d'écoulement au niveau de l'ouverture de sortie (25) environ 8 fois par minute à environ 50 fois par minute.

6. Générateur d'aérosol selon l'une des revendications 1 à 5,
**caractérisé en ce que** le dispositif de transport d'air (50) est conçu pour transporter, à chaque cycle de travail cyclique, un volume d'air d'environ 600 ml à environ 4000 ml ou d'environ 1500 ml à environ 3000 ml ou d'environ 700 ml à environ 1500 ml.

7. Générateur d'aérosol selon l'une des revendications 1 à 6,
**caractérisé en ce que** le dispositif de transport d'air (50) est conçu pour générer dans l'ouverture de sortie (25) une vitesse d'écoulement maximale d'environ 0,5 m/s à environ 2 m/s ou d'environ 0,8 m/s à environ 1,8 m/s ou d'environ 1,3 m/s à environ 1,5 m/s.

8. Procédé de distribution d'un aérosol dans un local, comprenant les étapes suivantes consistant à :
fournir un réservoir (40) avec un liquide (10),
produire un aérosol (15) à partir du liquide (10) au moyen d'un dispositif de nébulisation (45),
distribuer l'aérosol (15) dans le local par une première conduite (21) et une ouverture de sortie (25),
**caractérisé en ce que**
le sens d'écoulement est inversé de manière cyclique au niveau de l'ouverture de sortie (25) du fait qu'un dispositif de transport d'air (50), relié à la première conduite (21) par l'intermédiaire d'au moins une deuxième conduite (22), transporte et aspire de l'air de manière cyclique, un premier clapet anti-retour (31) étant prévu dans la deuxième conduite (22).

9. Procédé selon la revendication 8,
**caractérisé en ce que** l'aérosol (15) est produit à partir du liquide (10) au moyen d'un nébuliseur piézoélectrique ou d'un nébuliseur à buse ou d'un nébuliseur à membrane.

10. Procédé selon l'une des revendications 8 ou 9,
**caractérisé en ce que** le sens d'écoulement est inversé de manière cyclique au niveau de l'ouverture de sortie (25) environ 8 fois par minute à environ 50 fois par minute.

11. Procédé selon l'une des revendications 8 à 10,
**caractérisé en ce qu'**à chaque cycle de travail cyclique, un volume d'air d'environ 600 ml à environ 4000 ml ou d'environ 1500 ml à environ 3000 ml ou d'environ 700 ml à environ 1500 ml est transporté à travers l'ouverture de sortie (25).

12. Procédé selon l'une des revendications 8 à 11,
**caractérisé en ce qu'**une vitesse d'écoulement maximale d'environ 0,5 m/s à environ 2 m/s ou d'environ 0,8 m/s à environ 1,8 m/s ou d'environ 1,3 m/s à environ 1,7 m/s est générée dans l'ouverture de sortie (25).

13. Procédé selon l'une des revendications 8 à 12,
**caractérisé en ce que** le liquide (10) contenu dans le réservoir (40) contient ou est constitué d'eau ou d'une huile ou d'un liposome, et/ou **en ce que** le liquide (10) contenu dans le réservoir (40) contient ou est constitué d'une solution tampon.

14. Procédé selon la revendication 13,
**caractérisé en ce que** le liquide (10) dans le réservoir (40) contient des bactériophages.
